# EUROPEAN PATENT SPECIFICATION

(11) **EP 3 274 449 B1**
(45) Date of publication and mention of the grant of the patent: **04.03.2020**
(21) Application number: 16728750.7
(22) Date of filing: 24.03.2016
(51) Int. Cl.: C12N 9/12

(54) **MODIFIED 6-PHOSPHOFRUCTO-1 -KINASES, WHICH ENABLE FREMENTATIVE GROWTH OF RECOMBINANT YEAST SACCHAROMYCES CEREVISIAE CELLS ON PENTOSE SUGARS**
MODIFIZIERTE 6-PHOSPHOFRUCTO-1-KINASEN ZUR ERMÖGLICHUNG VON FERMENTATIVEM WACHSTUM VON REKOMBINANTEN SACCHAROMYCES-CEREVISIAE-HEFEZELLEN AUF PENTOSEZUCKERN
6-PHOSPHOFRUCTO-1-KINASES MODIFIÉES QUI PERMETTENT LA CROISSANCE PAR FERMENTATION DE CELLULES RECOMBINÉES DE LA LEVURE SACCHAROMYCES CEREVISIAE SUR DES PENTOSES

(30) Priority: 25.03.2015 SI 201500074
(43) Date of publication of application: 31.01.2018
(73) Proprietor: Kemijski Institut, 1001 Ljubljana (SI)
(72) Inventor: LEGISA, Matic, Medvode (SI); DRAGAN, Marko, Novo mesto (SI); ANDREJC, Darjan, Sostanj (SI); DOLINAR, Klemen, Ursna sela (SI); MOZIR, Alenka, Tolmin (SI)
(74) Representative: Flak, Antonija
(86) International application number: PCT/SI2016/000010
(87) International publication number: WO 2016/153437

(56) References cited:
- WO-A2-2007/123498
- CAPUDER MAJA ET AL: "Highly active, citrate inhibition resistant form of Aspergillus niger 6-phosphofructo-1-kinase encoded by a modified pfkA gene", JOURNAL OF BIOTECHNOLOGY, vol. 144, no. 1, Sp. Iss. SI, October 2009 (2009-10), pages 51-57, XP002762466, ISSN: 0168-1656
- SMERC ANDREJA ET AL: "Posttranslational modification of 6-phosphofructo-1-kinase as an important feature of cancer metabolism.", PLOS ONE, vol. 6, no. 5, E19645, May 2011 (2011-05), pages 1-12, XP002762467, ISSN: 1932-6203
- ANDREJC DARJAN ET AL: "Deregulated glycolytic flux in Saccharomyces cerevisiae", YEAST, vol. 30, no. Suppl. 1, September 2013 (2013-09), page S202, XP009191899, & 26TH INTERNATIONAL CONFERENCE ON YEAST GENETICS AND MOLECULAR BIOLOGY; FRANKFURT MAIN, GERMANY; AUGUST 29 -SEPTEMBER 03, 2013 ISSN: 0749-503X
- DATABASE ENA [Online] 4 August 2005 (2005-08-04), Mural et al.: "Mus musculus (house mouse) phosphofructokinase, muscle, isoform CRA_b", XP002762469, Database accession no. EDL04208 -& DATABASE UniParc [Online] 7 May 2007 (2007-05-07), XP002762930, Database accession no. UPI0001549DCB
- LEGISA MATIC ET AL: "Deregulation of the glycolytic flux at the level of 6-phosphofructo-1-kinase enables fermentative use of some pentose sugars by Saccharomyces cerevisiae", YEAST, vol. 32, no. Suppl. 1, September 2015 (2015-09), pages S156-S157, XP009191890, & 27TH INTERNATIONAL CONFERENCE ON YEAST GENETICS AND MOLECULAR BIOLOGY (ICYGMB); LEVICO TERME, ITALY; SEPTEMBER 06 -12, 2015 ISSN: 0749-503X

## Description

### The field of the invention

The subject of the invention is a modified glycolytic enzyme 6-phosphofructo-1-kinase (PFK) that enables fermentative growth of the recombinant yeast *Saccharomyces cerevisiae* cells on pentose sugars. The invention belongs to the field of genetic engineering of genes and microbial cells and fermentation processes for synthesis of specific chemical compounds, more specifically it belongs to the field of products formation that is possible by expression of modified genes in a host cell.

### Background of the invention and the technical problem

Contemporary technologies largely depend on discoveries and exploitation of fossil fuels, but they have numerous negative effects. At the start of the 21^{st} century a need for alternative energy sources has been formed that has mainly concentrated on plant biomass transformation into various fuels that could be used for transport. One of them is bio-ethanol production from sustainable sources.

Bio-ethanol can be easily produced from glucose extracted form corn and sugar cane, two industrial plants that are grown in limited amounts in Europe, and cannot meet all the requirements for bio-ethanol. For the second generation of bioethanol production, lignocellulose materials functioning as a raw material have been chosen that are abundant in Europe. They are found as a waste of the agricultural and the wood processing industry. Apart from glucose, lignocellulose materials contain various pentose sugars that are released after the hydrolysis of hemicellulose. The most abundant pentose sugar is xylose, while other less common pentose sugars are arabinose, xylulose, xylitol, ramnose, ribose and ribulose.

Important factor for the efficient and cheap bio-ethanol production form lignocellulose substrates are high productivities and yields of fermentations. Some microbial species were found to be the most important bio-catalizators, because they are easy to grow and can be genetically modified. Microorganisms that can perform such reactions must have the following characteristics:
- they must ferment pentose sugars,
- they must be resistant to different plant inhibitors,
- they must be resistant to high ethanol concentrations, and
- they must grow under the strict anaerobic and micro-anaerobic conditions
The yeast *Saccharomyces cerevisiae* is an excellent bio-ethanol producer. It can ferment glucose, mannose, fructose and galactose, however it is unable to grow on pentose sugars. Although the yeast *S. cerevisae* cannot ferment or assimilate xylose, some other yeast species can. In an extensive screening of 200 yeast strains able to grow on xylose, 19 were found that produced 0,1-1,0 g/L of ethanol under fermentative conditions from 20 g/L xylose (Toivola et al. 1984, Appl Environ Microbiol 47:1221-1223).

The yeast *S. cerevisiae* is a widely used model organism for numerous applications in the production of bio-ethanol, mainly due to its high resistance towards the high ethanol levels. Therefore, it is often genetically engineered for efficient bio-ethanol production from different plant hydrolysates. One of its best advantages in respect to some other yeast strains is its ability to accumulate higher yields of ethanol, since the wild type strain shows a tolerance up to approximately 12% (v/v) of ethanol, while the industrial strains exhibit even higher ethanol tolerance. Besides, yeast *S. cerevisiae* can theoretically convert up to 90-93% of glucose into the ethanol. On the other hand, the yeast *S. cerevisiae* possesses all the genes needed for the conversion of xylose into the ethanol (Bai et al. Biotechnol Adv 26, 1: 89-105). These enzymes are: xylose reductase (EC1.1.1.21), xylitol dehydrogenase(EC 1.1.1.9), xylulokinase (EC 2.7.1.17). However those genes are expressed at too low level to enable efficient assimilation of xylose (Richard et al., 1999, FEBS Letts 457, 1: 135-138). Besides, efficient metabolic conversion is hindered also by the specific NADH or NADPH oxido-reductase hat can unbalance fragile redox ratio in the yeast cells (van Maris et al., 2006, Antonie van Leuvenhoek 90: 391-418).

One of the main goals of the efficient bio-ethanol production from lignocellulose materials is therefore the construction of genetically engineered yeast *S. cerevisae* strains.

Pentose sugars can be efficiently transformed also into other fermentative products, apart from the bio-ethanol. One of them is 2-phenylethanol, a higher aromatic alcohol that is also a fragrance compound with a rose like odour. It is extremely popular in the cosmetics and perfume industry. Minor amounts are used also in the food industry as flavour compositions for food such as soft drinks and sweets. 2-phenylethanol is important as a raw material for the synthesis of derivatives such as esters. Particularly phenylacetate as a derivative is a valuable flavour and fragrance compound. Although 2-phenylethanol is a natural product, it is mostly produced by chemical synthesis (Etschmann et al., 2002, Appl Microbiol Biotechnol 59: 1-8). Yet, the flavours and fragrances that can be labelled "natural" both in the United States and Europe have to be produced from natural sources by physical, enzymatic or microbial processes (US Food and Drug Administration 2001).

The technical problem that is solved by the proposed invention is a modification of glycolytic genes that would enable fermentative use of pentose sugars and production of fermentative products by recombinant yeast cells. The invention should enable the use of genetically modified *S.cerevisiae* cells that would enable low cost, high efficiency product formation while the products themselves should be as pure as possible.

Besides, the solution of the technical problem should allow fast transition from the experimental level to the complex industrial level.

### State of the Art

So far, the technical problem is being solved by the insertion of heterologous genes for the degradation of pentose sugars in the yeast *S. cerevisiae.* Additionally the metabolic flux through pentose phosphate pathway was enhanced by the overexpression of homologous yeast genes.

In order to enable and enhance pentose sugars utilization by yeast *S. cerevisiae,* genetically modified cells have been engineered. In past a lot of effort was concentrated to the insertion of heterologous genes encoding the early part of xylose and arabinose assimilation toward D-xylulose-5-phosphate. Xylose fermentation in *S. cerevisae* could be achieved by expressing the *xy*/1 gene encoding xylose reductase (XR) and *xy*/2 gene for xylitol dehydrogenase (XDH). Both genes originate from the yeast *Pichia stipitis,* that can fermentatively grow on xylose (Koetter and Ciricy, 1993, Appl Microbiol Biotechnol 38, 6: 776-783). XR enzyme can be NADH or NADPH specific while XDH enzyme is only NADH specific. The use of both heterologous enzymes caused severe imbalance in NADH/NADPH ratio. One example to approach the redox factor imbalance was the expression of *gdp*1 gene from *Kluyveromyces lactis* encoding NADPH specific glyceraldehyde-3-phosphate dehydrogenase (GDP1). The enzyme allows NADPH production on expense of NADH production at the lower part of glycolysis (Verho et al., 2003, Appl Environ Microbiol 69, 109: 5892-5897). There were numerous other attempts to increase the conversion rate of xylose assimilation to xylitol and further to xylulose-5-phosphate that were nicely described by Passoth (2014, Molecular Mechanisms of Yeast Carbon Metabolism, 217-259).

Document FR2959514 describes the construction of an industrial yeast *S. cerevisiae* strain that includes: (i) selection of the host strain; (ii) integration of the expression cassette into the yeast genome; (iii) induced expression of at least one gene of each step of the pentose phosphate pathway; (iv) deletion of at least two copies of the *GRE3* gene of the yeast *S. cerevisiae.* The before mentioned cassette (ii) can be:
a) Expression cassette with the combination of ORF of the XRm gene from yeast *P.stipitis* that encodes mutated enzyme xylose reductase that is NADH specific instead of NADPH specific; and whose expression is controlled by the yeast *S. cerevisiae* promoter and terminator; and the cassette can be inserted into the genome.
b) Expression cassette with the combination of ORF of the gene XDH from yeast *P.stipitis* that encodes xylitol dehydrogenase; and whose expression is controlled by the yeast *S. cerevisiae* promoter and terminator; and the cassette can be inserted into the genome.
c) Expression cassette with the combination of ORF of the *XKS*1 gene from the yeast *S. cerevisiae* that encodes xylulokinase; and whose expression is controlled by the yeast *S. cerevisiae* promoter and terminator; and the cassette can be inserted into the genome.

Document KR20140067947 describes the *Pichia guilliermondii* Y-2 strain that is stored under the number KCTC 12322BP, a mixture of the strain culture is used for ethanol production. This document also describes the process for production of ethanol. The before mentioned strain ferments hexoses and pentoses, due to the presence of an enzyme that is a part of the xylose catabolism.

Document KR20130027984 discloses use of a mutated beta-glucosidase for bio-ethanol production.

Patent application US2012282664 describes a method for ethanol production by the yeast that metabolise xylose which is enabled by the insertion of a gene encoding an enzyme of the pentose phosphate pathway that originates from any other appropriate yeast species.

Patent application CA2834053 describes genetically modified yeast that contains exogene genes encoding pyruvate-formate-lyase and acetaldehyde dehydrogenase. Additionally, the yeast cells contain genetic modifications that allow better glycerol consumption such as increased NAD⁺-specific glycerol dehydrogenase activities and preferably one or two dihydroxyacetone kinase activities that make glycerol transport into the cell easier. The yeast cells also possess functional exogenic gene for xylose isomerase and/or functional genes that enable transition of L-arabinose to D-xylulose-5-phosphate. These genes can be genetically modified so that the synthesis of acetyl-CoA is increased. Such modified yeast cells ferment hexoses, pentoses, acetic acid into glycerol and ethanol.

In a patent application US2014308724 a yeast strain is described that can ferment pentose sugars from biomass, such as xylose and arabinose, into bioethanol.

Document FR2974114 describes ethanol production from cellulose and/or lignocellulose. The invention is based on the use of different micro-organisms preferably wild type *Pichis stipitis* or wild type *Candida shehatae,* for fermenting pentose sugars. Besides, other species were listed to be used for this purpose;
- bacteria from the genus *Bacillus, Bacteroides, Clostridium, Thermoanaerobacter,*
- recombinant bacteria *Escherichia coli, Klebsiella oxytoca, Zymomonas mobilis*
- yeast wild type species *Pachysolen tannophilus, Candida guilliermondii, Candida tropicalis*
- recombinant yeast *S. cerevisiae* as it was described before:
   ∘ Olsson in Hahn-Hägerdahl,1996, Enzyme Microb Technol 18: 312-331,
   ∘ Hahn-Hägerdahl et al., 2007, Adv Biochem Eng Biotechnol. 108:147-77,
   ∘ Öhgren et al., 2006, J Biotechnol 126(4): 488-98
- fungi from the genus *Fusarium*

Document KR20120118787 describes a yeast *P*. *stipitis* strain for bio-ethanol production that transforms xylose into ethanol. The strain is deficient in glucose receptor and possesses one of the following vectors: recombinant vector expressing reverse RNA against the glucose receptor that shows high affinity; and a recombinant vector with a methanol induced promoter that controls the expression of the gene encoding the glucose receptor.

Document US2012270290 describes bio-ethanol production from pentoses that originate from biomass. Document describes the use of a gene or protein that acts as a xylose or L-arabinose transporter. The gene belongs to a group where the HGT2, XUT1 and HXT2.4 genes are. The method for bio-ethanol production is based on the use of the yeast recombinants that contain at least one before mentioned gene while as a substrate biomass is taken that contains xylose and L-arabinose.

The second bottle neck in the xylose conversion to ethanol is located at the pentose phosphate (PP) pathway in the yeast *S. cerevisiae* cells. It begins with the D-xylulose-5-phosphate and ends with its conversion to fructose-6-phosphate and glyceraldehyde-3-phopshate, two molecules that enter the glycolytic pathway. With an aim to enhance metabolic flow through PP pathway, the expression of the following enzymes was increased: transaldolase (TAL), transketolase (TKL), ribose-5-phosphate isomerase (RKI) and ribulose-5-phosphate epimerase (RPE) (Karhumaa et al. 2005, Yeast 22: 359-368). By the enhanced metabolic flow through the PP pathway, growth of *S.cerevisiae* on xylose was established and no other genetic modifications were needed such as inserting the genes for enabling xylose conversion into D-xylulo-5-phosphate. These results confirm the thesis that efficient transduction of xylulose into D-xylulose-5-phopshate is needed in combination with the enhanced metabolic flow through the PP pathway.

The third, yet undescribed constraint in xylose conversion to ethanol may be present in the glycolytic flux. Two intermediates formed after the conversion of the xylose enter the glycolysis: fructose-6-phosphate at the level of 6-phosphofructo-1-kinase (PFK1) and glyceraldehyde-3-phosphate at the level of glyceraldehyde-3-phopshate dehydrogenase (GDP1). The key regulatory enzyme of glycolytic flux is PFK1 that enables more complex control over the flux than other two glycolytic regulatory enzymes: hexokinase and pyruvate kinase. PFK1 catalyses phosphorylation of fructose-1-phosphate (F6P) into fructose-1,6-bisphosphate at expense of the Mg-ATP as a phosphoryl donor. PFK1 is stimulated by fructose-2,6-bisphosphate (F-2,6-BP), ADP/AMP and ammonium ions, whereas citrate and ATP act as strong inhibitors (Dunaway, 1983, Mol Cell Biochem 52: 71-91). During the evolution, eukaryotic PFK1 enzymes developed by duplication, tandem fusion and divergence of catalytic and effector binding sites of a prokaryotic ancestor. However, the strict conservation between active site residues in the N-terminal ⁅ segment of the eukarytic enzyme and those of bacterial PFK1 enzymes suggest that the active site of eukaryotic PFKs is located only at the N-terminal portion (Poorman at al., 1984, Nature 309: 467-469).

A posttranslational modification of PFK1 has been described first in a filamentous fungus *Aspegillus niger* in an article by Mesojednik and Legiša (2005, Appl Environ Microbiol 71 (3): 1425-1432) as well as by Capuder et al. (2009, J Biotechnol., 144, 1, 51-57). Modification is caused by a proteolytic cleavage the C-terminal portion of the enzyme, so that a 49 kDa fragment is formed from the 85 kDa native enzyme. Newly formed enzyme is inactive initially but is activated after the phosphorylation of a threonine residue. The newly formed shorter fragments show changed enzymatic kinetics. Shorter PFK1 fragments have higher activities toward the substrate (F6P) while the activators such as F-2,6-BP and ammonium ions increase its activity more intensely than the activity of the native PFK1 enzyme. This article is silent about growth of the fungus on pentose sugars. On the other hand no inhibition of the shorter fragment can be detected by citrate while the ATP inhibition is prevented in the presence of physiological concentrations of F-2,6-BP (Mesojednik and Legiša 2005, Appl Environ Microbiol 71 (3): 1425-1432; Mlakar and Legiša 2007 Appl Environ Microbiol 72: 4515-4532). It seems that the formation of a highly active shorter PFK1 fragments deregulates glycolytic flux in the fungus *Aspergillus niger* and concomitantly induces citrate accumulation in the medium.

Similar posttranslational modification of PFK1 was later described in human cancer cells, more specifically at human muscle type PFK-M. In mammalian genomes three different PFK1 genes are present and are differently expressed in individual tissues. Apart from the muscle PFK-M type there is also liver PFK-L and platelet PFK-P type. Similarly to *A. niger* cells posttranslational modification of human PFK-M native enzyme was triggered by a proteolytic cleavage of the C-terminal part of the enzyme to form 47 kDa shorter fragments, however the human shorter fragments remained active and no phosphorylation of a specific threonine residue was needed (Smerc et al 2011, PLoS One 6, 5, e19645). This article is silent about inserting the short fragments of PFK into cells of *S. cerevisiae,* as well as ability of cells to use pentose sugars. Posttranslational modification of PFK1 enzyme might be the pivotal factor of deregulated glycolytic flux in tumours which is known as a Warburg effect. Undisturbed metabolic flow through the initial part of primary metabolism (glycolysis) is characterized by increased consumption of glucose in respect to the normal cells while the majority of glucose is transformed into lactate and excreted out of the cells. In the cancer cells up-regulated glycolysis takes place even in the presence of ample oxygen to fuel mitochondrial respiration. Deregulation of the *S. cerevisiae* metabolic pathway by shorter fragments of PFK has been presented by Andrejc et al. at a conference (Yeast, 30, Suppl. 1, ISSN: 0749-503X).

Patent application WO2007123498 discloses shorter PFK enzymes and genes encoding them, which may be inserted by means of an expression vector into filamentous fungi, bacteria or yeast, but does not disclose that the *S. cerevisiae* cells are able to use pentose sugars as carbon source based on insertion of these enzymes or genes.

In order to avoid complex posttranslational modification, the highly active shorter fragments can be synthesized also from the modified genes that are inserted into the recipient cells in an appropriate vector. To prepare a modified mt-*pfk*A gene of *A. niger,* specific mutation was introduced that prevented the need for phosphorylation of the newly formed shorter fragment for activation. Contrary to *A. niger* the modified mt-*pfk*A gene, the human modified sf *PFKM* gene encoding human highly active shorter PFK-M fragments were simply prepared by appropriate truncation of the native gene.

As described in the patent US 7,807,404 and EP 2 0101 651 increased productivity of specific metabolites of biotechnological value was recorded in several commercial microorganisms after the insertion of mt-*pfk*A gene of *A. niger.* Later tests revealed that the patented gene was instable since the synthesized protein contained a large unstructured intrinsic region.

The shorter PFK1 fragments originating from *A. niger* are unstable and consequently the recombinant cells with the inserted mt-*pfk*A gene cannot function properly. Therefore, the goal of the present invention is a stable PFK1 fragment that will be used in an industrial recombinant yeast cell that will be able to ferment pentose sugars and convert them into appropriate fermentative products. So far, no modifications describing regulatory glycolytic enzymes that would increase or enable pentose sugar assimilation have been published.

### Description of the invention

The subject of the invention is a yeast *Saccharomyces cerevisiae* cell comprising a fragment of enzyme 6-phopshofructo-1-kinase (PFK) encoded by a modified gene . The gene can be of human, animal and microbial origin or even a hybrid. This enzyme that is a key regulatory enzyme of glycolysis and consequently determines the rate of metabolic flow through the glycolysis as an initial part of primary metabolism. As a result of the modified gene expression, the active shorter PFK fragments are formed in the recombinant cells that show higher activities in respect to the native enzyme. Active shorter fragments in a combination with NADPH specific cytosolic malic enzyme encoded by *maeA* gene, which is also inserted into and expressed by the yeast S. *cerevisiae* cells enable growth of the yeast cells on pentose sugars and production of fermentative products, particularly 2-phenyethanol. Growth of recombinant cells and fermentative product accumulation can be further enhanced by the optimisation of growth conditions, especially by presence of a suitable nitrogen source.

In continuation, the invention will be further described by a detailed description and by using the following figures:
- Figure 1a:: Enzyme activities of the native human PFK-M enzyme at the increasing concentrations of substrate fructose-6-phophate and different concentrations of fructose-2,6-bisphosphate as a potential activator of PFK enzymes.
- Figure 1b:: Enzyme activities of the shorter human sf PFK-M fragments at increasing concentrations of substrate fructose-6-phophate and different concentrations of fructose-2,6-bisphosphate as a potential activator of PFK enzymes.
- Figure 2:: Growth of the recombinant yeast cells with the sf PFK-M shorter fragments and heterologous malic enzyme in the Sc*-ura⁻*, *leu⁻* medium that contains: 0,05% xylytiol; YNBQ; 5 mM glutamic acid; 10 µM FeSO₄. Wild type *S. cerevisae* strain and transformants with the native PFK-M enzyme with added malic enzyme as well as the transformants without malic enzyme didn't grow on the medium under the semi-anaerobic conditions.
- Figure 3:: Growth of the recombinant yeast cells with the sf PFK-M shorter fragments and heterologous malic enzyme under the semi-anaerobic and anaerobic conditions in the Sc*-ura⁻*, *leu⁻* medium that contains: 0,05% xylitol; YNB; 5 mM glutamic acid; 10 µM FeSO₄.
- Figure 4:: Growth of the recombinant yeast cells with the sf PFK-M shorter fragments and heterologous malic enzyme under the semi-anaerobic conditions in the Sc*-ura⁻*, *leu⁻* medium with YNB, 5 mM glutamic acid, 10 µM FeSO₄ with different sugars: xylose, xylitol and maltose. As a control the medium without sugar has been taken.
- Figure 5:: Growth of the recombinant yeast cells with the sf PFK-M shorter fragments and heterologous malic enzyme under the semi-anaerobic conditions in the Sc*-ura⁻*, *leu⁻* medium that contains: 0,05% xylitol; YNB; 10 µM FeSO₄ with different nitrogen sources: 5mM glutamine, 5 mM glutamic acid or 5g/L ammonium sulfate.
- Figure 6:: Growth of the recombinant yeast cells with the sf PFK-M shorter fragments and heterologous malic enzyme under the semi-anaerobic conditions at different initial concentrations of glutamic acid in the Sc*-ura⁻*, *leu⁻* medium that contains: 0,05% xylitol; YNB; 10 µM FeSO₄.
- Figure 7:: Xylitol consumption and 2-phenyethanol production during the growth of the recombinant yeast cells and heterologous malic enzyme in the Sc*-ura⁻*, *leu⁻* medium that contains: 0,05% xylitol; YNB; 5 mM glutamic acid; 10 µM FeSO₄
- Figure 8:: Growth of the recombinant yeast cells with the ssf PFK-M shorter fragments and with or without heterologous malic enzyme in the Sc-*ura⁻, leu⁻* medium that contains: 0,05% xylitol; YNB; 5 mM glutamic acid; 10 µM FeSO₄.
- Figure 9:: Growth of the recombinant yeast cells with the hybrid HBsf PFK-A and shorter mouse msf PFK-M fragment with or without heterologous malic enzyme in the Sc*-ura⁻*, *leu⁻* medium that contains: 0,05% xylitol; YNB; 5 mM glutamic acid; 10 µM FeSO₄.

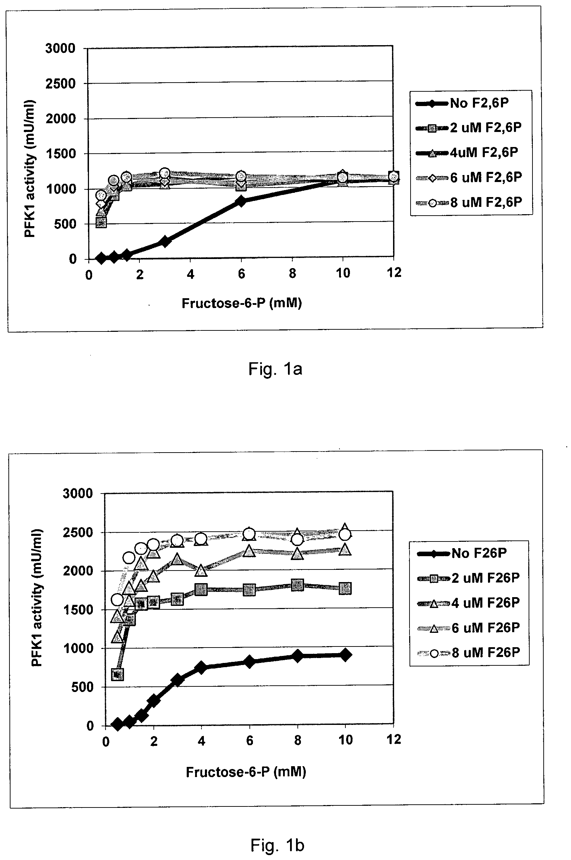

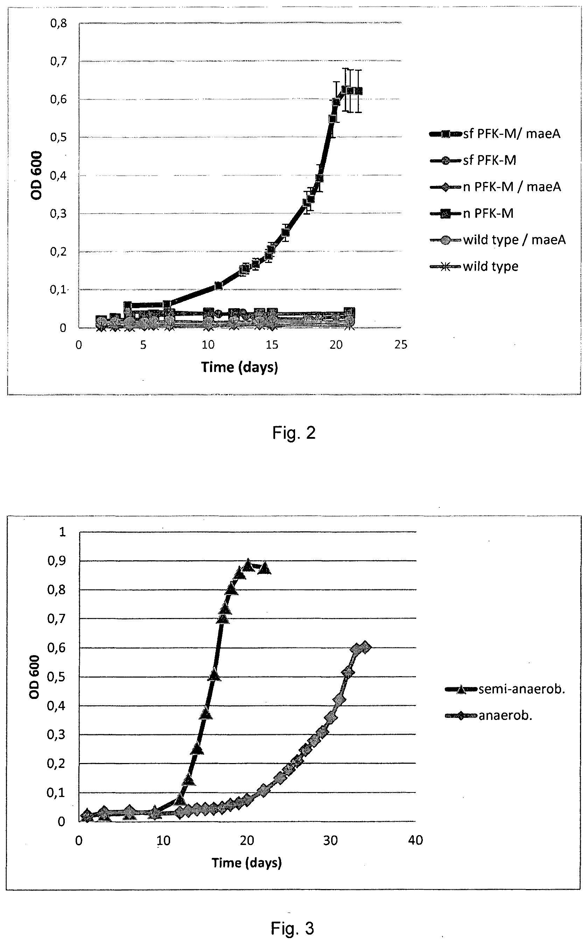

By determining kinetic characteristics of the partially purified native human PFK-M enzyme, we noticed that fructose-2,6-bisphosphate at the physiological concentrations increases the affinity of the enzyme toward the substrate. However, the kinetic characteristics of the shorter PFK-M fragment showed that fructo-2,6-phosphate increases the affinity and maximum velocity of the modified enzyme in respect to the activity of the enzyme measured without added activator. As shown in Fig.1 and Fig.2, the shorter PFK-M fragments are much more active at lower, physiological fuctose-6-phosphate concentrations than the native PFK-M enzyme. Similar kinetic characteristics were determined also with the native and shorter PFK1 enzymes from *A. niger.* Yet, highly active human shorter fragments encoded by the heterologous human sf-*pfk*-M genes have caused severe NADH/NADPH ratio imbalance during the growth of yeast cells in a media with fermentable sugars such as glucose and fructose.

The yeast *S. cerevisiae* does not possess transhydrogenase, the enzyme that can catalyse the conversion of NADH to NADPH and vice versa, and is present in other microorganisms (dos Santos et al., 2004, Metab Eng 6: 352-363). Moreover, it doesn't contain cytosolic NADPH specific malic enzyme that is present in the human cells. Deregulated glycolytic flux in human cancer cells was proposed to be triggered by the formation of highly active shorter PFK-M fragments. Due to the increased glucose consumption and enhanced metabolic flow, more NADH is formed that must be reoxidized by the reduction of pyruvate to lactate, and finally the former molecule is excreted out of the cells (Warburg effect). Enhanced glycolytic flux decreases the glucose assimilation through the pentose phosphate pathway that is an important source of NADPH. This reduced nucleotide is needed as a reducing power carrier that is indispensable in anabolic reactions. In human cancer cells imbalanced NADH/NADPH ratio is alleviated by the activity of the recombinant cytosolic NADPH specific malic enzyme, which converts malate into lactate. Malate is formed in mitochondria via tri-carboxylic acid (TCA) cycle and is excreted into cytosol. Due to the fact that about 85% of glucose is transformed into lactate in cancer cells, the TCA cycle is replenished through the glutamine which is the most abundant amino acid residue in the human serum. Glutamine, after the deamination to glutamate, enters the TCA cycle at the level of α-ketoglutarate. It has been shown that cancer cells can't grow in the medium without glutamine. (DeBerardinis et al., 2007, PNAS, 49: 19345-19350).

A similar system found in the cancer cells can be applied for the balancing the NADH/NADPH ratio in the yeast cells. It can be done by the insertion of the *mae*A gene encoding cytosolic NADP⁺ specific malic enzyme, the enzyme that converts malate into lactate while the released electrons reduce NADP⁺ into NADPH.

The present invention solves the technical problem by the modification of glycolytic enzymes. Recombinant yeast *S. cerevisae* transformants are able to run fermentative growth on some pentose sugars, and enable the formation of specific products. It is related to the use of modified PFK enzyme as well as the modified gene encoding the modified enzyme that is of microbial, animal or human origin. The solution of the technical problem is divided into the design of *S. cerevisiae* cells comprising the modified PFK enzymes with amino acid sequence selected in the group of amino acid sequences SEQ ID NO: 2 to SEQ ID NO: 5, and the gene encoding the malic enzyme as well as growth of transformants. More detailed solutions are described with examples.

### Construction of the gene encoding 6-phosphofructo-1-kinase fragments and the gene encoding malic enzyme

Two centromeric plasmids p416 and p415 have been used as vectors for the recombinant gene insertion into the yeast cells (Mumberg et al., 1995, Gene 156: 119-122). Both plasmids are low copy number plasmids. While p416 contains *ura*3 gene that complements *ura*3-52 auxotrophic mutation, plasmid p415 contains *leu*2 gene that complements leu2-3 auxotrophy. The expression of the inserted genes was under the control of GPD promoter. As a vector any other convenient plasmid can be used while the gene expression can be under the control of any other suitable constitutive or inducible promoter.

After all constructs were prepared, nucleotide sequences of the inserted genes were tested by sequencing.

### Construction of the native and modified human genes

The gene encoding human muscle type 6-phosphofructo-1-kinase PFK-M (cDNA Clone ID2964710) was purchased at Geneservice Ltd (Cambridge, UK). Human native gene designated n *pfk*M encoding 85 kDa native PFK-M protein, and gene designated sf *pfk*M encoding 47 kDa shorter sf PFK-M fragment were constructed as reported previously (Smerc et al., 2011, PLoS One 6 (5):e19645). Modified sf *pfk*M gene encoding the shorter PFK-M fragment with 443 amino acid residues was initially made for studying the Warburg effect in cancer cells, however later it was found that it was active and stable also in the *S.cerevisiae* cells and that the transformant may be used for fermentative product formation. By the use of polymerase chain reaction (PCR), new restriction sites were introduced at 5' (*XbaI*) and 3' (*BamHI*) ends that enabled ligation into the p416 plasmid. As a forward primer for the PCR reaction 5'-GAATTATCTAGAGCCACCATGACCCATGAAGAGCACCATGC-3' was used while 5'-TAATTCGGATCCTTACAGGCCCTCGAAACCATC-3' was used as a reverse primer for the sf *pfk*M and 5'-TAATTCGGATCCTTACAGGCCCTCGAAACCATC-3' for the n *pfk*M*.* Another slightly shorter sf *pfk*M gene designated ssf *pfk*M was constructed that encoded a shorter PFK-M fragment of 420 amino acid residues. For construction of this gene identical forward primer as for n *pfk*M and sf *pfk*M gene was used while 5'-TAATTCGGATCCTTAGCGAACAGCAGCATTCATGC-3' was used as a reverse primer.

Amino acid sequence of the native enzyme encoded by the n *pfk*M gene has been named as SEQ ID NO: 1; amino acid sequence encoded by the sf *pfk*M gene has been named SEQ ID NO: 2; amino acid sequence encoded by the ssf *pfk*M gene has been named SEQ ID NO: 3. The invention covers also the PFK-M fragments between 420 and 443 amino acid residues with added one or several consecutive amino acid residues of the STVRIGLIQGNRVLVVHDGFEG sequence that is added at the C-terminus of the amino acid residue sequence SEQ ID NO: 3. Because identical amino acid residues can be encoded by different DNA sequences, due to the different codon usage, all specified amino acid sequences that are encoded by different nucleotide sequences and nucleic acids are also protected by this invention.

### Construction of the modified mouse gene

A gene encoding native muscle PFK-M enzyme (cDNA C53048H23) was purchased at Source BioScience (Nottingham, UK). Shorter mouse gene designated msf *pfk*M encoding 47 kDa shorter msf PFK-M fragment has been constructed similarly as the human sf *pfk*M gene. Two restriction sides (*EcoRI* and *XBaI*) were inserted by the PCR reaction using forward primer 5'-GAATTAGAATTCATGACCCATGAAGAGCACTC-3' and reverse primer 5'-AATTATTCTAGATTACAGGACCCTCGAAACCATC-3'. Amino acid sequence of msf PFK-M enzyme was named SEQ ID NO: 4. Because identical amino acid residues can be coded by different DNA sequences, due to different codon usage, all specified amino acid sequences that are cover by different nucleotide sequences and nucleic acids are also protected by this invention.

### Construction of a hybrid gene

High instability of the highly active PFK1 fragment encoded by the *A. niger* mt-*pfkA* gene (Capuder et al., 2009, J Biotechnol 114: 51-57) has been revealed by the running the Predictions of Natural Disorder Regions (PONDR) algorithm. Therefore, we decided to stabilise a part of the internally disordered region of the fungal protein by replacing it with the more stable corresponding part of the human PFK-M enzyme. All together 99 amino acid residues were replaced between 189^{th} and 287^{th} amino acid residues. Hybrid enzyme was expected to be more stable compared with the enzyme encoded by the mt-*pfkA* gene.

The construction of the hybrid HBsf *pfkA* gene was conducted in two steps. First, 5' and 3' regions of the gene encoding PFK1 fragment of *A. niger* and the internal sequence of the shorter human PFK-M fragments were synthesised by PCR. The following primers were used:
- forward primer for the 5' end of the mt*-pfkA* gene 5'-CCATCGCACGCATATGGCTCCCCCCCAAGCTCCC-3';
- reverse primer for the 5' end of the mt*-pfkA* gene 5'-GGTAGTGATGGCGTCGACGGCGTCACAGA TGCGAG-3';
- forward primer for the internal region of the sf *pfkM* gene 5'-CGCCGTCGACGCCATCACTACCACTGCCCAG-3';
- reverse primer of the sf *pfkM* gene 5'-CACACGGGTATCATATCCCAGACGCTTAACCACCAG-3';
- forward primer for the 3' end of the mt-*pfk*A gene 5'-GCGTCTGGGATATGATACCCGTGTGACTGTGTTGGG-3';
- reverse primer for the 3' end of the mt-*pfk*A gene 5'-TAGGCGTTTATCGCTGCTTCTAGAGGATCCTTACCCGGGATCATAG-3'.
In the second reaction all three amplified regions were combined and the whole hybrid gene was amplified using the following oligonucleotides:
- forward primer 5'-CCATCGCACGCATATGGCTCCCCCCCAAGCTCCC-3';
- reverse primer:
   5'-TAGGCGTTTATCGCTGCTTCTAGAGGATCCTTACCCGGGATCATAG-3'.

Final product of the second PCR reaction was subjected to restriction enzymes *NdeI* and *XbaI,* reaction products were separated by agarose electrophoresis and purified. Restriction products ware ligated into the pALTER-Ex1 plasmid after the plasmid was exposed to identical restriction enzymes. Newly formed plasmid was cloned in the *E.coli* cells.
Isolated plasmid holding the HBsf *pfk*A gene was prepared for the insertion into the p416 plasmid after the *BamHI* and *HindIII* restriction sites were introduced at the 5' and 3' sites respectively. The following primers were used:
- forward primer:
   5'-GAATTAGGATCCGCCACCATGGCTCCCCCCCAAGCTCCCG-3';
- reverse primer:
   5'-TAATTCAAGCTTTTACCCGGGATCATAGTGCCGGCACAG-3'.

Amino acid sequence of the hybrid HBsf *pfk*A was named SEQ ID NO: 5. Because identical amino acid residues can be coded by different DNA sequences, due to the different codon usage, all specified amino acid sequences that are cover by different nucleotide sequences and nucleic acids are also protected by this invention.

### Construction of the maeA gene encoding the malic enzyme

Gene *mae*A encoding the synthesis of the malic enzyme was constructed as described previously (Zelle et al., 2011, Appl Environ Microbiol 77(3): 732-738). As a template, *scf*A gene of *E.coli* that is also designated as *mae*A has been taken. Using the codon optimisation program (Integrated DNA Technologies) codon usage for the yeast expression was optimized on the bacterial *sfc*A gene (Gene bank gi:90111281). By a single point mutation Asp336Gly, NADH specificity of the enzyme was changed to NADPH specificity. Synthetic gene was ordered in a form of gBLOCK at Integrated DNA Technologies (IDT, USA). Nucleotide sequence of the synthetic gene is listed under the name SEQ ID NO: 6 and the amino acid sequence under the name SEQ ID NO: 7. By the PCR reaction and by the use of specific primers, restriction sites were introduced to the gene that enabled insertion into the p415 plasmid. The following primers were used:
- forward primer
   5'-TAATTCGGATCCGCCACCATGGAACCTAAGACTAAGAAGCAACG-3', for the BamHI insertion
- reverse primer
   5'-TTCCTCGAGTTAAATACTTGTCCTACGATAGTCCCTATATTCGGCTTGCC-3' for the *Xba*I insertion

Amplified gene was inserted into the p415 plasmid that has *leu*-2 gene and complements leucine auxotrophy (*leu2-3*)*.*

### Construction of vectors with differently modified pfkM genes and maeA gene

The genes encoding different PFK-M forms and the gene encoding the malic enzyme were individually inserted into the appropriate vectors that can be any vector that enables the expression of the genes in the hosting cells of a microbial, plant or animal origin, preferably in the yeast *S. cerevisiae* cells. Vector must have a promoter, controlling sequences and other sequences that enable the expression. Preferably p416 plasmid was used for the modified genes and p415 plasmid for the *maeA* gene. The genes were inserted into the vector by the restriction and ligation methods that were described in standard protocols. In general, the procedure was conducted in the following steps:
- Restriction of modified *pfkM* gene or *maeA* gene by the appropriate restriction enzymes as it was previously described;
- Restriction of the vectors with identical restriction enzymes as it was described for the modified *pfk*M genes and the *mae*A gene;
- Separation and purification of restriction products from the previous steps;
- Ligation of the restriction products from the previous steps.

### Preparation of the recombinant cells of the yeast S. cerevisiae

For the construction of recombinant cells that will include the nucleic acid encoding the PFK-M fragment or a vector with the nucleic acid encoding any PFK-M fragment as described above, any host cell can be used, of microbial, plant or animal origin, preferably yeast *S. cerevisae* cells due to its capability to produce fermentative products. For recombinant cells, any yeast *S*. *cerevisiae* strain can be used, preferentially *pfk* null strain HD-114-8D.

The yeast strain HD56-5A (*MAT*α *ura 3-52 leu 2-3, 112 his 3-11, 15 MAL SUC GAL*) was used as a wild- type strain, and its isogenic *pfk*1*, pfk*2 null derivative, HD114-8D (*MAT*α *pfk1::HIS3 pfk2::HIS3 ura 3-52 leu2-3, 112 his3-11, 15 MAL SUC GAL*)*,* was used as a recipient for recombinant human genes (Heinisch et al., 1996, J Biol Chem 271, 27: 15928-15933).

For the vector insertion into the recipient cells any of the appropriate methods can be used. Preferably it is conducted according to the protocol that is based on the use of lithium acetate (Gietz et al., 1992, Nucleic Acids Res, 20(6): 1425). Transformants were initially grown on the solid minimal medium with additives (Synthetic drop out medium without uracil (Y1501 Sigma) and/or leucine (Y2001 Sigma). As a carbon source 2% glycerol and 2% ethanol were used. As the N-source Yeast nitrogen base has been taken (Y1251 Sigma) with 5g/L of added ammonium sulfate.

For the growth of host cells with the inserted nucleic acid for the synthesis of the PFK-M fragments or a vector with such nucleic acid, the medium was used that contains:
- Appropriate minimal medium with additives
- Yeast nitrogen base
- 10 uM FeSO₄
- C-source, preferably any pentose sugar, preferably xylose of xylitol
- N-source, preferably glutamic acid

The cells are grown at the temperatures in the range between 25 to 35°C, preferably at 30°C.

### Growth of transformed yeast cells and the use of the pentose sugars during the fermentation

### Growth of transformed yeast cells

The transformants were grown under the semi-anaerobic conditions in a sterile polycarbonate 125 mL Erlenmeyer flask with a venting cover (Corning Cat,No. 434211). They contained 100 ml of medium that was stirred by a 4 cm long magnetic stirrer and incubated at 30°C. The media contained maltose, xylose or xylitol for the fermentative growth, adequate minimal medium supplement (synthetic drop out medium) and adequate yeast nitrogen base (Yeast Nitrogen Base), glutamine (Q) or glutamic acid (E) at specified concentrations. Into all media 10 uM FeSO₄ was added. After the inoculation and before incubation the oxygen was removed from the medium by blowing the medium with nitrogen gas for 1 minute. During the growth, the samples have been removed from the medium by a 10 cm sterile syringe that was inserted through the venting cap. Growth kinetics was monitored by measuring optical density (OD₆₀₀) with a spectrophotometer (Lambda 25, Perkin-Elmer, Boston, MA, USA). Dry weights were determined from the optical density data using a calibration curve. For the calibration curve, dry weights were determined after the cells were collected on dry, pre-weighted glass microfiber filters (GF/A), washed with deionized water and dried to a constant weight in an oven at 105°C. The growth rate coefficient (K') was determined by the linear trend line prediction of the logarithmic values (Ln) of the dry cell weight increase during the exponential growth phase and expressed as reciprocal hours (h⁻¹). Generation time (g) as a measure of time (h) can be obtained by the equation g = Ln2/K' .

### Xylitol concentration determination

Xylitol concentration in the medium was determined by enzymatic test (Megazyme, Wicklow, Ireland) that contained sorbitol dehydrogenase and diaforaze. Before the test, the samples of a known volume were lyophilised and re-suspended in an equal volume of 50 mM Hepes buffer pH 7.5. Formasane formation was recorder at 492 nm.

### Phenyl-ethanol measurements

Phenylethanol concentrations were determined by GC-MS analyses. GC-MS analyses were performed using a Hewlett Packard Agilent 6890N gas chromatograph (Agilent Technologies) equipped with a mass spectrometer (Hewlett Packard Agilent 5973 N, Agilent Technologies). A 30 m length × 0.25 mm internal diameter capillary column (DB-35 ms, Agilent Technologies) with a 0.25 µm film thickness was used for separation. Helium was used as the carrier gas at a constant flow rate of 0.9 mL/min. The split ratio was 5:1, the split flow rate was 4.4 mL/min, and the injector volume was 5 µL. The oven temperature program was as follows: 50°C (2 min) to 300°C (5 min) at 30°C/min. A calibration curve was generated for each analyzed compound using an authentic standard sourced from Sigma-Aldrich (St. Louis, MO, USA). Calibration curves that were used for determination of metabolite concentrations were prepared using standards purchased at Sigma.

The present invention will be further described with the following examples, however the invention is not limited by the presented examples.

### Example I

The first example describes the sf *pfk*M gene encoding the fragment of human PFK-M enzyme.

A modified truncated human gene designated sf *pfk*M was constructed encoding the active shorter sf PFK-M fragments with 443 amino acid residues. Amino acid sequence of the fragments is presented by SEQ ID NO:2.

The sf *pfk*M gene was inserted into the p416 plasmid and concomitantly into the *S.cerevisiae* HD114-8D *pfk* null strain as described before.

Growth of the wild type yeast *S.cerevisiae* and different transformants with the native PFK-M and the sf PFK-M on xylitol under the semi-anaerobic conditions.

The growth of transformant with the sf PFK-M was compared to the growth of the transformant with the human native PFK-M and the wild type yeast strain HD56-5A that contains its own yeast PFK1 enzymes. Before mentioned transformants were compared for the growth of transformants containing the previously mentioned genes in combination with the *mae*A gene encoding the malic enzyme. The growth in the medium under the growth conditions as described above was followed by measuring optical density by a spectrophotometer. As shown in the Figure 3 all strains grew to approximate OD₆₀₀ value of 0,05 when the growth stopped with an exception of the transformant with the sf *pfk*M and *mae*A gene. The growth rate of this strain gradually increased to the OD₆₀₀ value of 0.6 to 0.7 when it stopped. No growth of the stains with other PFK-M forms was detected on xylitol, regardless of the *mae*A gene presence or absence.

The growth of the transformant with the sf PFK-M and malic anzyme under the semi-anaerobic and anaerobic conditions.

A low oxygen level in the cultivation flask was needed for a better growth of the strain on xylitol (Figure 3). The growth was slower when the conditions were anaerobic, caused by Parafilm sealed membrane positioned over the venting cap of the Corning flask.

### The growth on different sugars

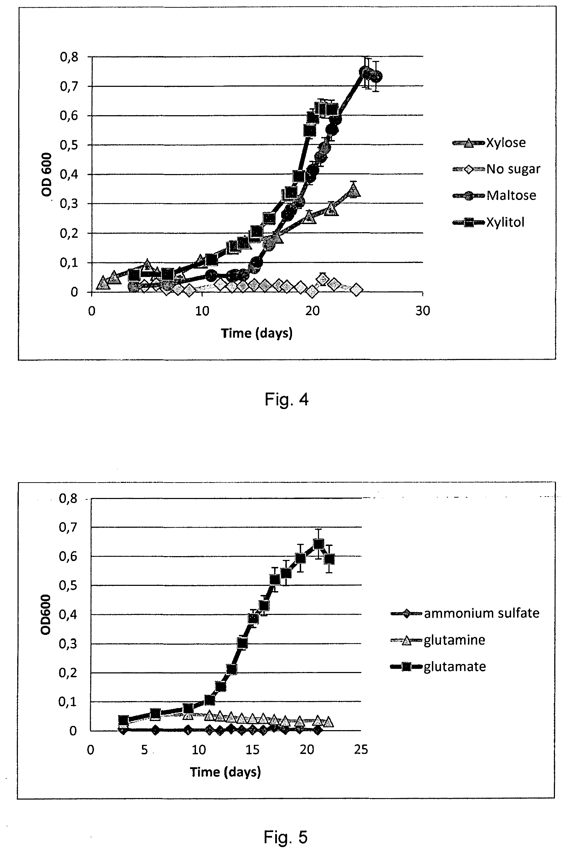

As shown in Figure 4 the strain with the sf PFK-M and malic enzyme grew on maltose and xylose as well. On maltose the lag phase was longer, however the specific growth rate was similar to that of the growth on xylitol while the yield was higher (OD₆₀₀ 0.7). In this experiment the sf *pfk*M/maeA transformant showed slightly shorter lag phase as during the experiment shown in Figure 4. The growth on xylose was significantly slower compared to xylitol, where the cell density reached the OD₆₀₀ value of only 0.35 after 25 days of fermentation. No growth was detected in the medium without added sugars even after 25 days.

The growth of the transformant with sf PFK-M fragment and the malic enzyme on xylitol and different nitrogen sources.

As shown in Figure 5 only glutamic acid enabled growth of the sf PFKM maeA transformant on xylitol. In the presence of glutamine the cells proliferated to the OD₆₀₀ value of 0.05, however the growth stopped later. With ammonium sulfate (5/L) in the medium, no growth was recorded and not even the value of 0.05 has been reached that was typically detected in the media containing the N-source in a form of amino acids.

The role of glutamic acid during the growth on xylitol.

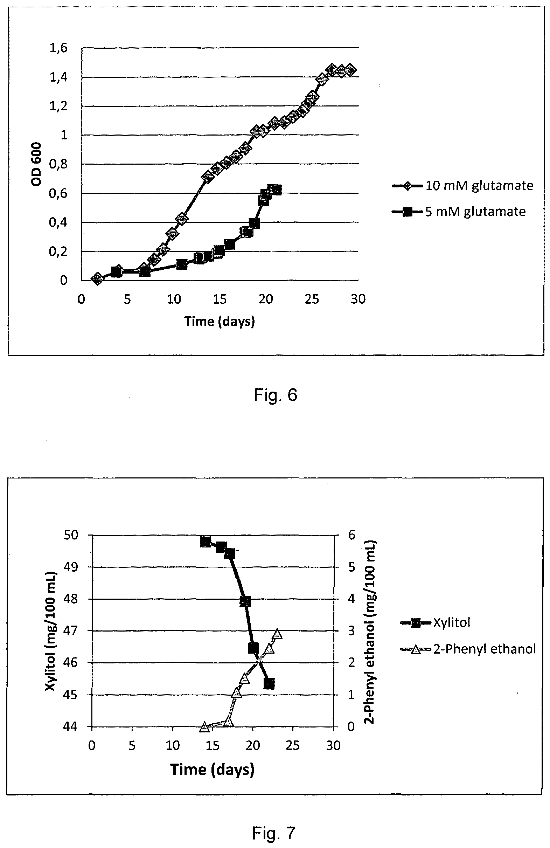

The importance of glutamic acid for the growth of the sf PFK-M *mae*A transformant on xylitol is shown in Figure 6. With the initial concentrations of 5 mM of glutamic acid and 0.05% xylitol, the culture proliferated to OD₆₀₀ value of 0.6 while with the initial concentration of 10 mM of glutamic acid and 0.05% of xylitol the final OD₆₀₀ value was 1.4. The *pfk* null HD-114-8D strain with inserted *mae*A gene didn't grow on 5 mM glutamic acid / 0.05% xylitol medium.

### Xylitol consumption and phenylethanol production of the sf pfkM maeA transformant

As shown in Figure 7, xylitol is minimally consumed during the growth of sf *pfk*M *mae*A transformant on the xylitol medium supplemented with glutamic acid. Xylitol dropped from the initial concentration of 50 mg/100 mL for only 5 mg. Meanwhile, no ethanol could be detected in the medium but 2-phenylethanol accumulated instead. During the fermentation under the semi-anaerobic conditions, about 3 mg of 2-phenylethanol was detected in the medium. Because no xylitol consumption or 2-phenyethanol formation could be detected during the growth of the *pfk* null HD118-8D strain with the inserted *mae*A gene, and because the sf *pfk*M *mae*A transformant didn't grow on the medium with glutamic acid with no added sugar, it is suggested that the majority of xylitol is converted to 2-phenylethanol by the sf *pfk*M *mae*A transformant.

### Example II

The second experiment deals with the additionally shortened human ssf *pfk*M gene that encodes a protein of 420 amino acid residues.

A modified truncated human gene designated ssf *pfk*M was constructed encoding the active shorter sf PFK-M fragments with 420 amino acid residues. Amino acid sequence of the fragment is presented by the SEQ ID NO: 3.
Modified ssf *pfk*M gene was inserted into the p416 plasmid and concomitantly into the *S.cerevisiae* HD144-8D *pfk* null strain as described before.

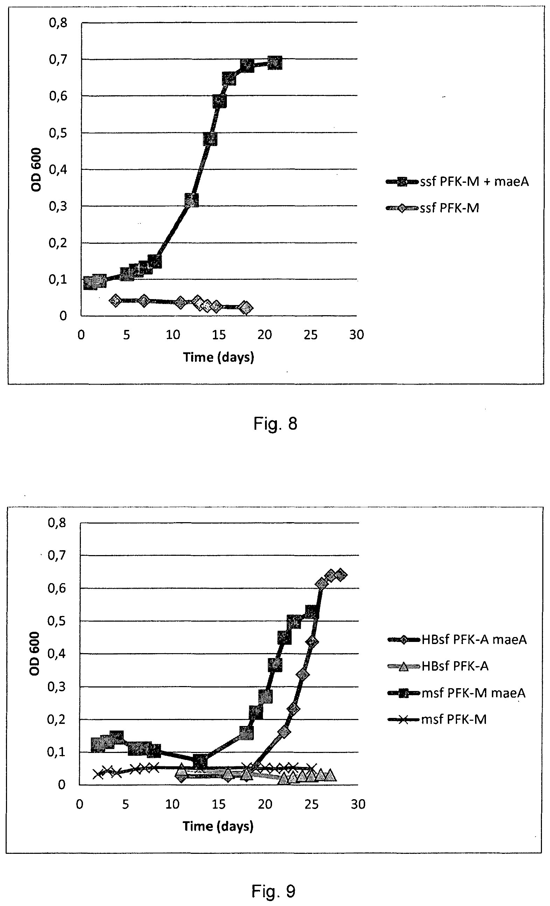

As shown in Figure 8, additionally shortened ssf PFK- fragments in combination with the malic enzyme enabled growth of the transformant on the xylitol. The growth rate of this transformants was slightly higher, reaching the growth rate coefficient K' 0,2891 (h⁻¹) in respect to the cells with the sf PFK-M fragments of 443 amino acid residues in combination with the malic enzyme (k' 0,2409). Also the ssf *pfk*M *maeA* transformat was able to consume xylitol and produce 2-phenylethanol.

### Example III

The third experiment describes msf *pfk*M gene encoding mouse shorter PFK-M fragment.

A modified truncated mouse gene designated msf *pfk*M was constructed encoding 47 kDa active shorter msf PFK-M fragments with 443 amino acid residues. Amino acid sequence of the fragment is presented by the SEQ ID NO: 4. The human and the mouse shorter fragments show high level of similarity, with 98,2% of homologous amino acid residues.

Modified ssf *pfk*M gene was inserted into the p416 plasmid and concomitantly into the *S.cerevisiae* HD144-8D *pfk* null strain as described before. Although the mouse fragment showed high level of homology to the human fragment, the later enabled higher rate of growth on a xylitol medium. Growth rate coefficient (k') of the strain with the human PFK-M and malic enzyme had a value of 0,2409 (h⁻¹) while the K' value of 0,1383 (h⁻¹) was recorded during the growth of the cells with mouse shorter PFK-M fragments and malic enzyme. The cells with the mouse shorter fragments showed similar xylitol consumption as the cells with the human shorter fragments and produced 2-phenylethanol.

### Example IV

The fourth experiment describes the hybrid HBsf *pfk*M gene encoding hybrid shorter PFK-M fragments.

A modified truncated hybrid gene designated HBsf *pfk*M was constructed encoding hybrid shorter fragment of *Aspergillus niger* with the replaced unstructured intrinsic region of 99 amino acid residues that originated from the human sf PFK-M enzyme. The amino acid sequence of this enzyme is presented by the SEQ ID NO: 5. Modified HBsf *pfk*M gene was inserted into the p416 plasmid and concomitantly into the *S.cerevisiae* HD144-8D *pfk* null strain as described before.

As shown in Figure 9 the yeast cells encoding the HBsf PFK-M enzyme and malic enzyme grew similarly as the cells with the human sf PFK-M enzyme and malic enzyme, which suggests they can use xylitol. During the growth of the HBsf PFK-M strain in the semi-anaerobic conditions, we detected slightly slower growth rate (K'0,2147 (h⁻¹) in respect to the transformant with the sf *pfk*M and *mae*A*.* The cells with the hybrid gene but without the gene encoding the malic enzyme were not able to grow on the xylitol during the 30 days long incubation.

The present invention deals with the adjusted modified genes encoding 6-phosphofructo-1-kinase (PFK) of microbial, animal or human origin that enables formation of the active shorter PFK fragments, that enable the growth of yeast *S. cerevisiae* transformants on xylitol of xylose. Viability of the cells is enabled by the expression of the gene encoding cytosolic malic enzyme that is inserted into the cells together with the gene encoding individual fragment. Under the semi-anaerobic conditions, the activity of malic enzyme is dependent on the presence of glutamic acid in the medium that acts a precursor for the synthesis of malate by the tri-carboxylic acid cycle. During the fermentative growth on xylitol or xylose, 2-phenylethanol is formed that presents the solution for the technical problem. The described invention may be used in applications that require pentose sugars metabolism, more specifically at processes for fermentative metabolite production, primarily for the bioethanol and/or 2-phenylethanol production.

### Sequence listing

SEQ ID NO: 1















SEQ ID NO: 2
MTHEEHHAAKTLGIGKAIAVLTSGGDAQGMNAAVRAVVRVGIFTGARVFFVHEGYQGL
VDGGDHIKEATWESVSMMLQLGGTVIGSARCKDFREREGRLRAAYNLVKRGITNLCVI
GGDGSLTGADTFRSEWSDLLSDLQKAGKITDEEATKSSYLNIVGLVGSIDNDFCGTDMT
IGTDSALHRIMEIVDAITTTAQSHQRTFVLEVMGRHCGYLALVTSLSCGADWVFIPECPP
DDDWEEHLCRRLSETRTRGSRLNIIIVAEGAIDKNGKPITSEDIKNLVVKRLGYDTRVTVL
GHVQRGGTPSAFDRILGSRMGVEAVMALLEGTPDTPACVVSLSGNQAVRLPLMECVQ
VTKDVTKAMDEKKFDEALKLRGRSFMNNWEVYKLLAHVRPPVSKSGSHTVAVMNVGA
PAAGMNAAVRSTVRIGLIQGNRVLVVHDGFEGL

SEQ ID NO: 3

MTHEEHHAAKTLGIGKAIAVLTSGGDAQGMNAAVRAVVRVGIFTGARVFFVHEGYQGL
VDGGDHIKEATWESVSMMLQLGGTVIGSARCKDFREREGRLRAAYNLVKRGITNLCVI
GGDGSLTGADTFRSEWSDLLSDLQKAGKITDEEATKSSYLNIVGLVGSIDNDFCGTDMT
IGTDSALHRIMEIVDAITTTAQSHQRTFVLEVMGRHCGYLALVTSLSCGADWVFIPECPP
DDDWEEHLCRRLSETRTRGSRLNIIIVAEGAIDKNGKPITSEDIKNLVVKRLGYDTRVTVL
GHVQRGGTPSAFDRILGSRMGVEAVMALLEGTPDTPACVVSLSGNQAVRLPLMECVQ
VTKDVTKAMDEKKFDEALKLRGRSFMNNWEVYKLLAHVRPPVSKSGSHTVAVMNVGA
PAAGMNAAVR

SEQ ID NO: 4
MTHEEHHAAKTLGIGKAIAVLTSGGDAQGMNAAVRAVVRVGIFTGARVFFVHEGYQGL
VDGGEHIREATWESVSMMLQLGGTVIGSARCKDFREREGRLRAAHNLVKRGITNLCVI
GGDGSLTGADTFRSEWSDLLNDLQKDGKITAEEATKSSYLNIVGLVGSIDNDFCGTDM
TIGTDSALHRIVEIVDAITTTAQSHQRTFVLEVMGRHCGYLALVTSLSCGADWVFIPECP
PDDDWEEHLCRRLSETRTRGSRLNIIIVAEGAIDKNGKPITSEDIKNLVVKRLGYDTRVT
VLGHVQRGGTPSAFDRILGSRMGVEAVMALLEGTPDTPACVVSLSGNQAVRLPLMEC
VQVTKDVTKAMDEKRFDEAIKLRGRSFMNNWEVYKLLAHVRPPVSKGGLHTVAVMNV
GAPAAGMNAAVRSTVRIGLIQGNRVLVVHDGFEGL

SEQ ID NO: 5
MAPPQAPVQPPKRRRIGVLTSGGDAPGMNGVVRAVVRMAIHSDCEAFAVYEGYEGLV
NGGDMIRQLHWEDVRGWLSRGGTLIGSARCMEFRERPGRLRAAKNMVLRGIDALVVC
GGDGSLTGADVFRSEWPGLLKELVETGELTEEQVKPYQILNIVGLVGSIDNDMSGTDAT
IGCYSSLTRICDAVDAITTTAQSHQRTFVLEVMGRHCGYLALVTSLSCGADWVFIPECP
PDDDWEEHLCRRLSETRTRGSRLNIIIVAEGAIDKNGKPITSEDIKNLVVKRLGYDTRVT
VLGHTQRGGAACAYDRWLSTLQGVEAVRAVLDMKPEAPSPVITIRENKILRMPLMDAV
QHTKTVTKHIQNKEFAEAMALRDSEFKEYHFSYINTSTPDHPKLLLPENKRMRIGIIHVG
APAGGMNQATRAAVAYCLTRGHTPLAIHNGFPGLCRHYD

SEQ ID NO: 6

ATGGAACCTAAGACTAAGAAGCAACGTTCTTTATACATTCCTTATGCTGGTCCAGTT
TTGTTGGAGTTCCCACTACTGAATAAAGGCTCAGCTTTTAGTATGGAGGAAAGGAG
AAATTTCAATCTGTTGGGCTTATTACCGGAAGTAGTCGAAACTATAGAAGAACAGGC
TGAAAGGGCATGGATTCAATATCAAGGCTTCAAGACTGAAATTGATAAGCACATTTA
CTTACGTAACATACAGGATACAAACGAAACACTTTTCTATAGATTGGTAAATAACCAT
TTAGATGAGATGATGCCTGTTATTTACACTCCTACTGTTGGCGCCGCTTGCGAGAG
ATTTAGTGAGATATACAGGAGAAGCAGAGGGGTCTTTATCTCCTATCAAAATAGACA
TAACATGGATGATATCTTACAAAACGTCCCCAACCACAATATTAAAGTTATTGTAGTT
ACTGATGGGGAAAGAATTCTAGGACTGGGTGACCAAGGCATAGGCGGTATGGGTA
TCCCAATTGGAAAACTTTCTTTATACACGGCATGCGGTGGTATTTCCCCTGCCTATA
CACTTCCAGTTGTGCTAGACGTGGGCACTAAT
AACCAACAGCTATTAAACGATCCTCTATATATGGGGTGGAGGAATCCTAGAATAACT
GACGATGAGTACTATGAATTTGTTGATGAATTTATTCAAGCTGTCAAGCAGAGATGG
CCCGATGTGTTGCTTCAATTTGAGGATTTCGCACAAAAAAATGCAATGCCATTATTA
AATCGTTACAGAAATGAAATTTGTTCATTTAACGATGATATTCAAGGAACCGCCGCC
GTTACCGTCGGTACATTAATTGCCGCGTCCAGAGCCGCGGGGGTCAGCTGTCTG
AAAAAAAGATTGTATTCCTTGGTGCCGGTTCAGCTGGCTGCGGCATCGCTGAAATG
ATTATCGCTCAAACACAAAGGGAAGGCTTAAGTGAAGAAGCTGCCAGGCAAAAAGT
TTTTATGGTTGGTAGATTCGGACTGCTTACTGACAAGATGCCAAATTTGTTGCCTTT
CCAAACCAAGTTAGTTCAGAAAAGGGAAAATTTATCTGACTGGGATACTGACTCCG
ACGTTCTTTCATTGCTTGACGTCGTTAGGAACGTTAAACCTGATATTTTGATTGGAG
TCTCAGGCCAAACCGGTCTATTTACCGAAGAAATT
ATAAGAGAGATGCACAAGCATTGTCCAAGACCCATCGTGATGCCGTTATCAAATCC
AACATCAAGGGTCGAAGCTACACCTCAGGATATTATTGCATGGACTGAAGGTAACG
CCTTAGTCGCTACCGGTTCTCCATTTAATCCTGTAGTCTGGAAGGACAAAATATATC
CGATAGCACAATGTAACAATGCTTTTATATTTCCAGGTATAGGATTGGGAGTGATTG
CCTCTGGTGCGTCCAGAATAACAGACGAAATGCTAATGTCCGCTAGTGAAACACTG
GCCCAATACAGTCCATTAGTTTTAAATGGAGAGGGCTTGGTTCTTCCTGAACTTAAG
GATATACAGAAAGTGAGTAGAGCGATCGCATTTGCAGTCGGTAAGATGGCTCAACA

ACAGGGCGTAGCCGTAAAAACCAGTGCAGAAGCTCTGCAACAGGCTATAGATGAC
AATTTTTGGCAAGCCGAATATAGGGACTATCGTAGGACAAGTATTTAA

SEQ ID NO: 7
MEPKTKKQRSLYIPYAGPVLLEFPLLNKGSAFSMEERRNFNLLGLLPEVVETIEEQAER
AWIQYQGFKTEIDKHIYLRNIQDTNETLFYRLVNNHLDEMMPVIYTPTVGAACERFSEIY
RRSRGVFISYQNRHNMDDILQNVPNHNIKVIVVTDGERILGLGDQGIGGMGIPIGKLSLY
TACGGISPAYTLPVVLDVGTNNQQLLNDPLYMGWRNPRITDDEYYEFVDEFIQAVKQR
WPDVLLQFEDFAQKNAMPLLNRYRNEICSFNDDIQGTAAVTVGTLIAASRAAGGQLSEK
KIVFLGAGSAGCGIAEMIIAQTQREGLSEEAARQKVFMVGRFGLLTDKMPNLLPFQTKL
VQKRENLSDWDTDSDVLSLLDVVRNVKPDILIGVSGQTGLFTEEIIREMHKHCPRPIVMP
LSNPTSRVEATPQDIIAWTEGNALVATGSPFNPVVWKDKIYPIAQCNNAFIFPGIGLGVIA
SGASRITDEMLMSASETLAQYSPLVLNGEGLVLPELKDIQKVSRAIAFAVGKMAQQQGV
AVKTSAEALQQAIDDNFWQAEYRDYRRTSI

### Zaporedja

SEQ ID NO: 1















SEQ ID NO: 2
MTHEEHHAAKTLGIGKAIAVLTSGGDAQGMNAAVRAVVRVGIFTGARVFFVHEGYQ
GLVDGGDHIKEATWESVSMMLQLGGTVIGSARCKDFREREGRLRAAYNLVKRGITN
LCVIGGDGSLTGADTFRSEWSDLLSDLQKAGKITDEEATKSSYLNIVGLVGSIDNDF
CGTDMTIGTDSALHRIMEIVDAITTTAQSHQRTFVLEVMGRHCGYLALVTSLSCGAD
WVFIPECPPDDDWEEHLCRRLSETRTRGSRLNIIIVAEGAIDKNGKPITSEDIKNLVVK
RLGYDTRVTVLGHVQRGGTPSAFDRILGSRMGVEAVMALLEGTPDTPACVVSLSG
NQAVRLPLMECVQVTKDVTKAMDEKKFDEALKLRGRSFMNNWEVYKLLAHVRPPV
SKSGSHTVAVMNVGAPAAGMNAAVRSTVRIGLIQGNRVLVVHDGFEGL

SEQ ID NO: 3
MTHEEHHAAKTLGIGKAIAVLTSGGDAQGMNAAVRAVVRVGIFTGARVFFVHEGYQ
GLVDGGDHIKEATWESVSMMLQLGGTVIGSARCKDFREREGRLRAAYNLVKRGITN
LCVIGGDGSLTGADTFRSEWSDLLSDLQKAGKITDEEATKSSYLNIVGLVGSIDNDF

CGTDMTIGTDSALHRIMEIVDAITTTAQSHQRTFVLEVMGRHCGYLALVTSLSCGAD
WVFIPECPPDDDWEEHLCRRLSETRTRGSRLNIIIVAEGAIDKNGKPITSEDIKNLVVK
RLGYDTRVTVLGHVQRGGTPSAFDRILGSRMGVEAVMALLEGTPDTPACVVSLSG
NQAVRLPLMECVQVTKDVTKAMDEKKFDEALKLRGRSFMNNWEVYKLLAHVRPPV
SKSGSHTVAVMNVGAPAAGMNAAVR

SEQ ID NO: 4
MTHEEHHAAKTLGIGKAIAVLTSGGDAQGMNAAVRAVVRVGIFTGARVFFVHEGYQ
GLVDGGEHIREATWESVSMMLQLGGTVIGSARCKDFREREGRLRAAHNLVKRGITN
LCVIGGDGSLTGADTFRSEWSDLLNDLQKDGKITAEEATKSSYLNIVGLVGSIDNDF
CGTDMTIGTDSALHRIVEIVDAITTTAQSHQRTFVLEVMGRHCGYLALVTSLSCGAD
WVFIPECPPDDDWEEHLCRRLSETRTRGSRLNIIIVAEGAIDKNGKPITSEDIKNLVVK
RLGYDTRVTVLGHVQRGGTPSAFDRILGSRMGVEAVMALLEGTPDTPACVVSLSG
NQAVRLPLMECVQVTKDVTKAMDEKRFDEAIKLRGRSFMNNWEVYKLLAHVRPPV
SKGGLHTVAVMNVGAPAAGMNAAVRSTVRIGLIQGNRVLVVHDGFEGL

SEQ ID NO: 5
MAPPQAPVQPPKRRRIGVLTSGGDAPGMNGVVRAVVRMAIHSDCEAFAVYEGYEG
LVNGGDMIRQLHWEDVRGWLSRGGTLIGSARCMEFRERPGRLRAAKNMVLRGIDA
LVVCGGDGSLTGADVFRSEWPGLLKELVETGELTEEQVKPYQILNIVGLVGSIDNDM
SGTDATIGCYSSLTRICDAVDAITTTAQSHQRTFVLEVMGRHCGYLALVTSLSCGAD
WVFIPECPPDDDWEEHLCRRLSETRTRGSRLNIIIVAEGAIDKNGKPITSEDIKNLVVK
RLGYDTRVTVLGHTQRGGAACAYDRWLSTLQGVEAVRAVLDMKPEAPSPVITIREN
KILRMPLMDAVQHTKTVTKHIQNKEFAEAMALRDSEFKEYHFSYINTSTPDHPKLLLP
ENKRMRIGIIHVGAPAGGMNQATRAAVAYCLTRGHTPLAIHNGFPGLCRHYD

SEQ ID NO: 6
ATGGAACCTAAGACTAAGAAGCAACGTTCTTTATACATTCCTTATGCTGGTCCAG
TTTTGTTGGAGTTCCCACTACTGAATAAAGGCTCAGCTTTTAGTATGGAGGAAAG
GAGAAATTTCAATCTGTTGGGCTTATTACCGGAAGTAGTCGAAACTATAGAAGAA
CAGGCTGAAAGGGCATGGATTCAATATCAAGGCTTCAAGACTGAAATTGATAAG
CACATTTACTTACGTAACATACAGGATACAAACGAAACACTTTTCTATAGATTGGT

AAATAACCATTTAGATGAGATGATGCCTGTTATTTACACTCCTACTGTTGGCGCC
GCTTGCGAGAGATTTAGTGAGATATACAGGAGAAGCAGAGGGGTCTTTATCTCC
TATCAAAATAGACATAACATGGATGATATCTTACAAAACGTCCCCAACCACAATA
TTAAAGTTATTGTAGTTACTGATGGGGAAAGAATTCTAGGACTGGGTGACCAAG
GCATAGGCGGTATGGGTATCCCAATTGGAAAACTTTCTTTATACACGGCATGCG
GTGGTATTTCCCCTGCCTATACACTTCCAGTTGTGCTAGACGTGGGCACTAAT
AACCAACAGCTATTAAACGATCCTCTATATATGGGGTGGAGGAATCCTAGAATAA
CTGACGATGAGTACTATGAATTTGTTGATGAATTTATTCAAGCTGTCAAGCAGAG
ATGGCCCGATGTGTTGCTTCAATTTGAGGATTTCGCACAAAAAAATGCAATGCCA
TTATTAAATCGTTACAGAAATGAAATTTGTTCATTTAACGATGATATTCAAGGAAC
CGCCGCCGTTACCGTCGGTACATTAATTGCCGCGTCCAGAGCCGCGGGGGGTC
AGCTGTCTGAAAAAAAGATTGTATTCCTTGGTGCCGGTTCAGCTGGCTGCGGCA
TCGCTGAAATGATTATCGCTCAAACACAAAGGGAAGGCTTAAGTGAAGAAGCTG
CCAGGCAAAAAGTTTTTATGGTTGGTAGATTCGGACTGCTTACTGACAAGATGC
CAAATTTGTTGCCTTTCCAAACCAAGTTAGTTCAGAAAAGGAAAATTTATCTGA
CTGGGATACTGACTCCGACGTTCTTTCATTGCTTGACGTCGTTAGGAACGTTAAA
CCTGATATTTTGATTGGAGTCTCAGGCCAAACCGGTCTATTTACCGAAGAAATT
ATAAGAGAGATGCACAAGCATTGTCCAAGACCCATCGTGATGCCGTTATCAAAT
CCAACATCAAGGGTCGAAGCTACACCTCAGGATATTATTGCATGGACTGAAGGT
AACGCCTTAGTCGCTACCGGTTCTCCATTTAATCCTGTAGTCTGGAAGGACAAA
ATATATCCGATAGCACAATGTAACAATGCTTTTATATTTCCAGGTATAGGATTGG
GAGTGATTGCCTCTGGTGCGTCCAGAATAACAGACGAAATGCTAATGTCCGCTA
GTGAAACACTGGCCCAATACAGTCCATTAGTTTTAAATGGAGAGGGCTTGGTTC
TTCCTGAACTTAAGGATATACAGAAAGTGAGTAGAGCGATCGCATTTGCAGTCG
GTAAGATGGCTCAACAACAGGGCGTAGCCGTAAAAACCAGTGCAGAAGCTCTG
CAACAGGCTATAGATGACAATTTTTGGCAAGCCGAATATAGGGACTATCGTAGG
ACAAGTATTTAA

SEQ ID NO: 7
MEPKTKKQRSLYIPYAGPVLLEFPLLNKGSAFSMEERRNFNLLGLLPEVVETIEEQA
ERAWIQYQGFKTEIDKHIYLRNIQDTNETLFYRLVNNHLDEMMPVIYTPTVGAACERF
SEIYRRSRGVFISYQNRHNMDDILQNVPNHNIKVIVVTDGERILGLGDQGIGGMGIPI

GKLSLYTACGGISPAYTLPVVLDVGTNNQQLLNDPLYMGWRNPRITDDEYYEFVDE
FIQAVKQRWPDVLLQFEDFAQKNAMPLLNRYRNEICSFNDDIQGTAAVTVGTLIAAS
RAAGGQLSEKKIVFLGAGSAGCGIAEMIIAQTQREGLSEEAARQKVFMVGRFGLLTD
KMPNLLPFQTKLVQKRENLSDWDTDSDVLSLLDVVRNVKPDILIGVSGQTGLFTEEII
REMHKHCPRPIVMPLSNPTSRVEATPQDIIAWTEGNALVATGSPFNPVVWKDKIYPI
AQCNNAFIFPGIGLGVIASGASRITDEMLMSASETLAQYSPLVLNGEGLVLPELKDIQ
KVSRAIAFAVGKMAQQQGVAVKTSAEALQQAIDDNFWQAEYRDYRRTSI

## Claims

1. A yeast *Saccharomyces cerevisiae* cell comprising a fragment of enzyme 6-phosphofructo-1-kinase, which is of microbial, animal or human origin, and has amino acid sequence selected in a group consisting of sequences SEQ ID NO: 2, SEQ ID NO: 3, SEQ ID NO: 4, SEQ ID NO: 5 and sequences that after the SEQ ID NO: 3 contain one or more consecutive amino acids from the amino acid sequence STVRIGLIQGNRVLVVHDGFEG, **characterized in that** it further comprises and expresses *mae*A gene, which encodes a cytosolic malic enzyme, and that the yeast cell is able to utilize pentose sugars as carbon source while using a suitable nitrogen source.

2. A hybrid fragment of enzyme 6-phosphofructo-1-kinase composed of enzymatic parts of the microbial and human origin having an amino acid sequence SEQ ID NO: 5.

3. A yeast *Saccharomyces cerevisiae* cell comprising the hybrid fragment of enzyme 6-phoshofructo-1-kinase according to claim 2, further comprising and expressing *mae*A gene, which encodes a cytosolic malic enzyme, wherein the yeast cell is able to utilize pentose sugars as carbon source while using a suitable nitrogen source.

4. Yeast *Saccharomyces cerevisiae* cells according to the claims from 1 and 3, **characterised in that** the cytosolic NADPH specific malic enzyme has amino acid sequence SEQ ID NO: 7, which is encoded by *mae*A gene with a preferred nucleotide sequence SEQ ID NO: 6.

5. Yeast *Saccharomyces cerevisiae* cells according to any claim from 1, 3 or 4, **characterised in that** the pentose sugars are preferably xylitol and xylose.

6. Yeast *Saccharomyces cerevisiae* cells according to any claim from 1, 3 to 5, **characterised in that** the nitrogen source is glutamic acid or glutamate.

7. Use of yeast *Saccharomyces cerevisiae* cells or the hybrid fragment according to claims 1 to 6 in applications that include pentose catabolism.

8. Use of yeast *Saccharomyces cerevisiae* cells or the hybrid fragment according to claim 7, **characterised in that** the application is production of fermentative products, preferentially bio-ethanol or 2-phenylethanol.

## Patentansprüche

1. Hefezelle von *Saccharomyces cerevisiae,* umfassend ein Fragment des Enzyms 6-Phosphofructo-1-Kinase, das mikrobiellen, tierischen oder menschlichen Ursprungs ist, und eine Aminosäuresequenz aufweist, die aus einer Gruppe ausgewählt ist, die aus den Sequenzen SEQ ID NO: 2, SEQ ID NO: 3, SEQ ID NO: 4, SEQ ID NO: 5 besteht, und aus Sequenzen, die nach der SEQ ID NO: 3 eine oder mehrere aufeinanderfolgende Aminosäuren aus der Aminosäuresequenz STVRIGLIQGNRVLVVHDGFEG enthalten, **dadurch gekennzeichnet, dass** sie außerdem das, das zytosolische Malatenzym kodierentes maeA-Gen umfasst und exprimiert, und dass die Hefezelle unter Verwendung einer geeigneten Stickstoffquelle Pentosezucker als Kohlenstoffquelle nutzen kann.

2. Hybrid-Fragment des Enzyms 6-Phosphofructo-1-Kinase bestehend aus enzymatischen Teilen mikrobiellen und menschlichen Ursprungs mit einer Aminosäuresequenz SEQ ID NO: 5.

3. Hefezelle von *Saccharomyces cerevisiae,* umfassend ein Hybrid-Fragment des Enzyms 6-Phosphofructo-1-Kinase nach Anspruch 2, ferner umfassend und exprimierend das, das zytosolische Malatenzym kodierentes maeA-Gen, wobei die Hefezelle in der Lage ist, Pentosezucker als Kohlenstoffquelle unter Verwendung einer geeigneten Stickstoffquelle zu nutzen.

4. Hefezelle von *Saccharomyces cerevisiae* nach Ansprüchen 1 und 3, **dadurch gekennzeichnet, dass** das zytosolische NADPH-spezifische Malatenzym die Aminosäuresequenz SEQ ID NO: 7 aufweist, die von dem maeA-Gen mit einer bevorzugten Nukleotidsequenz SEQ ID NO: 6 kodiert wird.

5. Hefezelle von *Saccharomyces cerevisiae* nach einem der Ansprüche 1, 3 oder 4, **dadurch gekennzeichnet, dass** die Pentosezucker bevorzugt Xylitol und Xylose sind.

6. Hefezelle von *Saccharomyces cerevisiae* nach einem der Ansprüche 1, 3 bis 5, **dadurch gekennzeichnet, dass** die Stickstoffquelle Glutaminsäure oder Glutamat ist.

7. Verwendung der Hefezelle von *Saccharomyces cerevisiae* oder des Hybrid-Fragments nach Ansprüchen 1 bis 6 in Anwendungen, die Pentose-Katabolismus beinhalten.

8. Verwendung der Hefezelle von *Saccharomyces cerevisiae* oder des Hybrid-Fragments nach Anspruch 7, **dadurch gekennzeichnet, dass** die Anwendung die Herstellung von fermentativen Produkten, vorzugsweise von Bio-Ethanol oder 2-Phenylethanol, ist.

## Revendications

1. Cellule de levure *Saccharomyces **cerevisiae*** comprenant un fragment d'enzyme 6-phosphofructo-1-kinase, qui est d'origine microbienne, animale ou humaine et a une séquence d'acides aminés choisie dans un groupe constitué par les séquences SEQ ID NO: 2, SEQ ID NO: 3, SEQ ID NO: 4, SEQ ID NO: 5 et des séquences qui, après la SEQ ID NO: 3, contiennent un ou plusieurs acides aminés consécutifs de la séquence d'acides aminés STVRIGLIQGNRVLVVHDGFEG, **caractérisée en ce qu'**en outre elle contient et exprime le gène *mae*A*,* qui code l'enzyme malique cytosolique, et **en ce que** la levure est capable d'utiliser des sucres pentoses comme source de carbone tout en utilisant une source d'azote adéquate.

2. Fragment hybride de l'enzyme 6-phosphofructo-1-kinase composé de parties d'enzyme d'origine microbienne ou humaine ayant la séquence d'acides aminés SEQ ID NO: 5.

3. Cellule de levure *Saccharomyces **cerevisiae*** comprenant le fragment de l'enzyme 6-phosphofructo-1-kinase selon la revendication 2, qui en outre comprend et exprime le gène *mae*A*,* qui code l'enzyme malique cytosolique, la cellule de levure étant capable d'utiliser des sucres pentoses comme source de carbone tout en utilisant une source d'azote adéquate.

4. Cellules de levure *Saccharomyces cerevisiae* selon les revendications 1 et 3, **caractérisées en ce que** l'enzyme malique cytosolique NADPH-spécifique possède la séquence d'acides aminés SEQ ID NO: 7, qui est codée par le gène *mae*A avec la séquence de nucléotides préférée SEQ ID NO: 6.

5. Cellules de levure *Saccharomyces **cerevisiae*** selon l'une quelconque des revendications 1, 3 ou 4, **caractérisées en ce que** les sucres pentoses sont de préférence le xylitol et le xylose.

6. Cellules de levure *Saccharomyces cerevisiae* selon l'une quelconque des revendications 1, 3 à 5, **caractérisées en ce que** la source d'azote est l'acide glutamique ou le glutamate.

7. Utilisation de cellules de levure *Saccharomyces cerevisiae* ou du fragment hybride selon les revendications 1 à 6 dans les applications qui incluent le catabolisme de pentose.

8. Utilisation de cellules de levure *Saccharomyces cerevisiae* ou du fragment hybride selon la revendication 7, **caractérisée en ce que** l'application est la production de produits fermentaires, de préférence de bioéthanol ou de 2-phényléthanol.
